# EUROPÄISCHE PATENTSCHRIFT

(11) **EP 1 567 514 B1**
(45) Veröffentlichungstag und Bekanntmachung des Hinweises auf die Patenterteilung: **23.07.2008**
(21) Anmeldenummer: 03811775.0
(22) Anmeldetag: 27.11.2003
(51) Int. Cl.: C07D 317/26, C07C 45/82, C07C 47/198

(54) **VERFAHREN ZUR GEWINNUNG EINES ALIPHATISCHEN DIALDEHYD-MONOACETALS**
METHOD FOR PRODUCING AN ALIPHATIC DIALDEHYDE MONOACETAL
PROCEDE DE PRODUCTION D'UN DIALDEHYDE MONOACETAL ALIPHATIQUE

(30) Priorität: 28.11.2002 DE 10255647
(43) Veröffentlichungstag der Anmeldung: 31.08.2005
(73) Patentinhaber: BASF SE, 67056 Ludwigshafen (DE)
(72) Erfinder: HADERLEIN, Gerd, 67269 Gründstadt (DE); GÖBBEL, Hans-Georg, 67169 Kallstadt (DE); SIEGEL, Wolfgang, 67117 Limburgerhof (DE)
(74) Vertreter: Isenbruck, Günter
(86) Internationale Anmeldenummer: PCT/EP2003/013370
(87) Internationale Veröffentlichungsnummer: WO 2004/048359

(56) Entgegenhaltungen:
- EP-A- 0 987 261

## Beschreibung

Die Erfindung betrifft ein Verfahren zur Gewinnung eines reinen aliphatischen Dialdehyd-Monoacetals.

Dialdehyde sind in der organischen Synthese, insbesondere von Pharmazeutika, Agrochemikalien sowie anderen Wirk- und Effektstoffen, aufgrund der Reaktivität und der vielfältigen Umsetzungsmöglichkeiten der Aldehydfunktionen wertvolle Synthesebausteine. Besonders interessant sind Dialdehyde, bei denen eine der beiden Aldehyd-Funktionen maskiert, d.h. geschützt ist. So lassen sich in der Synthese-Sequenz die beiden funktionellen Gruppen gezielt und getrennt durch jeweils geeignete Reaktionen und Entschützungen umsetzen.

Eine besonders einfache, gleichzeitig effektive und auch leicht wieder abzuspaltende Schutzgruppe ist das Aldehyd-Acetal. Daher stellen insbesondere aliphatische Dialdehyde, bei denen eine der beiden Aldehyd-Funktionen mit Alkoholen oder Thiolen acetalisiert ist, also Dialdehyd-Monoacetale sowie deren Substitutionsprodukte, interessante und wertvolle Intermediate in der organischen Synthese dar.

Im Übersichtsartikel von C. Botteghi und F. Soccolini in Synthesis 1985, Seiten 592 bis 604 sind verschiedene Synthesemöglichkeiten für Dialdehyd-Monoacetale der allgemeinen Formel 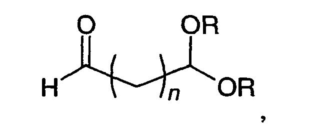 für *n* = 1, 2 oder 3, zusammengestellt.

Die beschriebenen Synthesewege sind jedoch für den großtechnischen Einsatz nicht geeignet.

Nach derzeitigem Kenntnisstand ist, insbesondere für die besonders interessanten Monoacetale des Glutaraldehyds, d.h. Verbindungen entsprechend der obigen allgemeinen Formel mit n = 3, der einzige, für den großtechnischen Maßstab wirtschaftliche Syntheseweg die direkte Umsetzung von Glutaraldehyd mit dem entsprechenden Alkohol.

Hierzu sind insbesondere die folgenden Varianten bekannt:

Nach dem Verfahren der JP 48-39416 wird Glutaraldehyd direkt säurekatalysiert mit Ethylenglykol im Verhältnis 2:1 umgesetzt. Das Verfahren liefert das Wertprodukt, das Monoethylenglykol-Acetal des Glutaraldehyds, 2-(3-Formylpropyl)-1,3-dioxolan, im Folgenden abgekürzt mit FPDO bezeichnet, nach Destillation in 40%iger Ausbeute. Der Überschuss an Glutaraldehyd muss jedoch destillativ abgetrennt werden.

Nach dem Verfahren der JP 48-61477 wird Glutaraldehyd mit einem Überschuss an Ethylenglykol zum Diacetal umgesetzt. Dieses wird dann nach Isolierung mit Wasser zum Monoacetal hydrolysiert. Nach extraktiver Aufreinigung erhält man das Wertprodukt FPDO in 48%iger Ausbeute.

Nach dem Verfahren der JP 11-228566 wird Glutaraldehyd zunächst mit Ethylenglykol ebenfalls zum Diacetal umgesetzt. Dieses wird dann jedoch nach Isolierung mit weiterem Glutaraldehyd zum Wertprodukt FPDO komproportioniert.

Aus EP-A-0 987 261 ist zwar das direkte, d.h. einstufige, Verfahren zur Herstellung von Dialdehyd-Monoacetalen bekannt, nach dem Kenntnisstand der EP-A-0 987 261 war jedoch davon auszugehen, dass auf Grund der Gleichgewichtsreaktion, wonach stets neben dem Monoacetal auch das entsprechende Diacetal gebildet wird, bei einer anschließenden (diskontinuierlichen) Destillation lediglich niedrige Ausbeuten an Monoacetal, von maximal 20%, erreicht werden können. Das Dokument schlägt daher ein wesentlich komplizierteres Verfahren vor, und zwar eine zweistufige Verfahrensführung, bei der in einer ersten Verfahrensstufe aus einem Dialdehyd und einem Diol das entsprechende Bisacetal gebildet wird, wonach Wasser und ein Teil des Diols abdestilliert werden. Das Bisacetal aus dem Kolonnensumpf wird durch Zugabe von weiterem Dialdehyd zum Monoacetal komproportioniert und destillativ abgetrennt. Hierbei handelt es sich um eine diskontinuierliche Verfahrensführung.

Die bekannten Verfahren haben insbesondere die folgenden Nachteile: Bei allen Reaktionen wird immer eine Mischung aus Edukten, dem Wertprodukt Monoacetal und Bisacetal gebildet. Bei den Verfahren der JP 48-61477 oder JP 11-228566, bei denen zunächst gezielt das Bisacetal hergestellt wird, liegt das Gleichgewicht in Bezug auf die Produkte zwar etwas günstiger. Trotzdem muss nach wie vor das Gemisch aufgetrennt werden; es wird jedoch zudem eine zusätzliche Verfahrensstufe benötigt.

Ein Problem aller bekannter Verfahren, das noch nicht in technisch und wirtschaftlich sinnvoller Weise gelöst ist, ist, dass aufgrund der hohen Reaktivität der beiden Aldehyd-Funktionen im Edukt Dialdehyd und teilweise maskiert im Produkt Dialdehyd-Monoacetal die Reaktionsmischungen, vor allem bei erhöhter Temperatur, leicht polymerisieren. Dies liefert schlecht zu handhabende, hochviskose Produkte und führt zu Ausbeuteverlusten. Insbesondere bei der destillativen Aufarbeitung als Batch-Destillation mit hohen Verweilzeiten bei hoher Temperatur, wie sie in den vorstehend beschriebenen Verfahren durchgeführt wurde, führt dies zu Produktverlusten.

Es war daher Aufgabe der Erfindung, ein Verfahren zur Verfügung zu stellen, das in wirtschaftlich vorteilhafter Weise einstufig zum Wertprodukt Dialdehyd-Monoacetal führt, mit einem hohen Reinheitsgrad von mindestens 98 Gew.-%, um die Spezifikationsanforderungen für den Einsatz in nachfolgenden Synthesen zu erfüllen, und bei dem die Produktverluste durch Polymerisation niedrig gehalten werden. Insbesondere bei der Destillation der Rohware sollen Produktverluste minimiert werden.

Die Aufgabe wird gelöst durch ein Verfahren zur Gewinnung eines reinen aliphatischen Dialdehyd-Monoacetals, das zu mindestens 98 Gew.-% aus dem Wertprodukt, aliphatisches Dialdehyd-Monoacetal, gebildet ist, durch Umsetzung des entsprechenden aliphatischen Dialdehyds oder eines Vorläufers des entsprechenden aliphatischen Dialdehyds mit einem oder mehreren aliphatischen ein- oder mehrwertigen Alkoholen, wobei man Wasser destillativ abtrennt unter Erhalt eines Reaktionsgemisches, das man destillativ auftrennt, das dadurch gekennzeichnet ist, dass man die destillative Auftrennung kontinuierlich in einer Trennwandkolonne durchführt, wobei man reines aliphatisches Dialdehyd-Monoacetal als Seitenabzug der Trennwandkolonne erhält oder in zwei Destillationskolonnen, wobei man in der ersten Destillationskolonne rohes aliphatisches Dialdehyd-Monoacetal als Seitenabzug erhält, das rohe aliphatische Dialdehyd-Monoacetal der zweiten Destillationskolonne zuführt und aus der zweiten Destillationskolonne reines aliphatisches Dialdehyd-Monoacetal als Seitenabzug erhält.

Als rohes aliphatisches Dialdehyd-Monoacetal wird vorliegend ein Gemisch bezeichnet, das zu mindestens 90 Gew.-%, bevorzugt zu mindestens 97 Gew.-% aus dem Wertprodukt, dem aliphatischen Dialdehyd-Monoacetal gebildet ist.

Als reines aliphatisches Dialdehyd-Monoacetal wird vorliegend ein Gemisch bezeichnet, das zu mindestens 98 Gew.-%, bevorzugt zu mindestens 99 Gew.-% aus dem Wertprodukt, aliphatisches Dialdehyd-Monoacetal, gebildet ist.

Die Erfindung ist nicht eingeschränkt bezüglich der konkreten Durchführung der Umsetzung des aliphatischen Dialdehyds oder eines Vorläufers des aliphatischen Dialdehyds mit einem oder mehreren aliphatischen ein- oder mehrwertigen Alkoholen.

In einer bevorzugten Variante wird der Dialdehyd, bevorzugt Glutaraldehyd, in wässriger Lösung, bevorzugt bis zu 50 Gew.-% in Wasser, vorgelegt und auf 30 bis 80°C, bevorzugt auf 40 bis 50°C, besonders bevorzugt auf 45°C vorerwärmt. Anschließend wird Vakuum angelegt, so dass das Lösungswasser abdestilliert. Gleichzeitig zum Abdestillieren des Wassers wird Alkohol oder eine Mischung von Alkoholen, bevorzugt Ethylenglykol, zugefahren. Gegen Ende der Umsetzung wird die Temperatur auf 50 bis 110°C, bevorzugt auf 80 bis 90°C, besonders bevorzugt auf 85°C erhöht.

In einer weiteren Verfahrensvariante kann der aliphatische Dialdehyd, bevorzugt Glutaraldehyd, der als wässrige Lösung vorliegt, unter Anlegen von Vakuum, bevorzugt bei geringfügig erhöhter Temperatur, im Bereich von 30 bis 80°C, bevorzugt bei 40 bis 50°C, besonders bevorzugt bei 45°C entwässert werden. Hierbei muss jedoch aufgrund der Tendenz zu spontaner Polymerisation darauf geachtet werden, dass der entwässerte Dialdehyd auf einer Temperatur im oben genannten Bereich sowie stets in Bewegung gehalten wird und unmittelbar nach der Entwässerung umgesetzt wird. Hierzu wird, analog zur Verfahrensvariante, der Alkohol oder die Mischung von Alkoholen, bevorzugt Ethylenglykol, zugefahren.

In einer weiteren Verfahrensvariante ist es möglich, beide Edukte, den Dialdehyd sowie den oder die Alkohole und gegebenenfalls den Katalysator vorzulegen, wobei man den Dialdehyd bevorzugt in wässriger Lösung einsetzt und anschließend sowohl das Lösungswasser als auch das Reaktionswasser abdestilliert. Bei dieser Ausführungsvariante ist allerdings die Raum-Zeit-Ausbeute gegenüber den vorstehend beschriebenen Varianten verschlechtert.

Als aliphatischer Dialdehyd kann bevorzugt eine Substanz aus der nachfolgenden Aufzählung eingesetzt werden: Malonaldehyd, Succinaldehyd, Glutaraldehyd oder Adipinaldehyd oder deren alkylsubstituierte Derivate, besonders bevorzugt Glutaraldehyd, insbesondere in wässriger Lösung, bevorzugt in 50 gew-%iger wässriger Lösung oder dessen Vorläufer, 2-Hydroxy-3,4-dihydo-2H-pyran.

Als Alkoholkomponente können insbesondere einwertige Alkohole, wie Methanol, Ethanol, n-Propanol, i-Propanol, n-Butanol, sec-Butanol, i-Butanol, oder Diole eingesetzt werden, insbesondere Ethylenglykol, 1,2-Propylenglykol, 1,3-Propylenglykol, 1,2-Butandiol, 1,3-Butandiol oder 1,4-Butandiol, wobei Ethylenglykol besonders bevorzugt ist.

Besonders bevorzugt setzt man Glutaraldehyd mit Ethylenglykol in einem Molverhältnis im Bereich von 1:1,5 bis 1,5:1, bevorzugt von 1:1,2 bis 1,2:1, insbesondere von 1,0:1,0 ein. Obwohl die Umsetzung zum aliphatischen Dialdehyd-Monoacetal auch unkatalysiert abläuft, ist der Einsatz eines sauren Katalysators bevorzugt, insbesondere eines Kationenaustauschers, einer Mineralsäure, bevorzugt Schwefelsäure, Chlorwasserstoffsäure, besonders bevorzugt Orthophosphorsäure oder einer organischen Säure, insbesondere Essigsäure, p-Toluolsulfonsäure oder Methansulfonsäure, in einer Konzentration von 0,02 bis 5 Gew.-%, bevorzugt von 0,1 bis 1 Gew.-%, besonders bevorzugt von 0,3 Gew.-%, bezogen auf das Gesamtgewicht der Reaktionsmischung.

Das durch Destillation von der Wasserfracht nahezu vollständig befreite Reaktionsgemisch wird zur Gewinnung des Wertproduktes, des aliphatischen Dialdehyd-Monoacetals, anschließend destillativ aufgetrennt.

Die Erfinder haben erkannt, dass es hierfür wesentlich ist, die Destillation kontinuierlich durchzuführen. Kontinuierlich betriebene Destillationen haben gegenüber den bekannten diskontinuierlich durchgeführten Destillationen den Vorteil der geringeren Verweilzeit des Sumpfproduktes und damit der geringeren thermischen Belastung bzw. Schädigung. Indem die Destillation erfindungsgemäß kontinuierlich durchgeführt wird, wird eine signifikante Verbesserung der Destillationsausbeute erreicht.

Die kontinuierliche destillative Auftrennung kann in zwei aufeinanderfolgenden Destillationskolonnen oder, besonders vorteilhaft, in einer Trennwandkolonne durchgeführt werden.

Für die Durchführung der Destillation in zwei aufeinanderfolgenden Destillationskolonnen wird das nahezu wasserfreie Reaktionsgemisch zunächst einer ersten Destillationskolonne, die vorteilhaft 40 bis 80 theoretische Trennstufen, bevorzugt 50 bis 70, besonders bevorzugt 60 bis 70 theoretische Trennstufen aufweist, zugeführt und bei einem Kopfdruck von 5 bis 500 mbar, bevorzugt von 10 bis 300 mbar, besonders bevorzugt von 15 bis 100 mbar, kontinuierlich destilliert.

Als Kopfprodukt wird nicht umgesetzter Glutaraldehyd abgetrennt und vorzugsweise in die Synthese recycliert. Aus dem Verstärkungsteil der Kolonne, d.h. oberhalb der Zuführung des aufzutrennenden Gemisches, wird über einen flüssigen Seitenabzug rohes aliphatisches Dialdehyd-Monoacetal abgezogen, d.h. ein Gemisch, das mindestens 90 Gew.-%, vorzugsweise mindestens 97 Gew.-% des Wertproduktes, Monoacetal, enthält.

Am Sumpf der Kolonne fallen das Diacetal sowie höher kondensierte Produkte an. Als Sumpfverdampfer eignen sich insbesondere Fallfilmverdampfer, da sie eine schonende Verdampfung garantieren und somit das thermisch empfindliche Produkt nicht belasten.

Bevorzugt wird der Sumpfaustrag der ersten Destillationskolonne in einem nachfolgenden Dünnschichtverdampfer bei einem Druck von vorzugsweise etwa 10 mbar in zwei Ströme aufgeteilt: das flüchtige Diacetal wird über Kopf abgetrennt, kondensiert und zur Rückspaltung in die Acetalisierungsstufe recycliert. Die schwersiedenden Polymere werden thermisch verwertet.

Das rohe aliphatische Dialdehyd-Monoacetal wird anschließend einer zweiten Destillationskolonne zugeführt, die vorzugsweise 30 bis 70 theoretische Trennstufen, insbesondere 40 bis 70, besonders bevorzugt 50 bis 70 theoretische Trennstufen aufweist und bei einem Kopfdruck 5 bis 500 mbar, bevorzugt von 10 bis 300 mbar, besonders bevorzugt von 15 bis 100 mbar, betrieben wird.

Als Kopfstrom wird aus der zweiten Destillationskolonne restlicher Dialdehyd abgetrennt und bevorzugt in die Synthese recycliert.

Aus dem Abtriebsteil der Kolonne, d.h. unterhalb der Zuführung des aufzutrennenden Gemisches in die zweite Destillationskolonne, wird als dampfförmiger Seitenabzug reines aliphatisches Dialdehyd-Monoacetal abgezogen, d.h. ein Gemisch, das mindestens 98 Gew.-% des Wertproduk-tes aliphatisches Dialdehyd-Monoacetal, bevorzugt 99 Gew.-% des Wertproduktes, enthält.

Am Sumpf der Kolonne fallen höhersiedende Komponenten an, die noch Anteile des Wertproduktes, aliphatisches Dialdehyd-Monoacetal, enthalten. Um den Wertstoffverlust zu reduzieren, wird der Sumpfstrom vorzugsweise in die erste Destillationskolonne recycliert.

In einer besonders vorteilhaften Verfahrensvariante wird die kontinuierliche Destillation in einem einzigen Apparat, einer Trennwandkolonne, durchgeführt.

Es ist bekannt, dass Trennwandkolonnen eine besonders wirtschaftliche, insbesondere bezüglich der Investitions- und Energiekosten günstige Auftrennung von Mehrkomponenten-Gemischen unter Erhalt eines oder mehrerer reiner Seitenströme ermöglichen. In Trennwandkolonnen ist in Teilbereichen derselben eine Quervermischung der Flüssigkeits- und Brüdenströme durch eine Trennwand, in der Regel ein in Kolonnenlängsrichtung angeordnetes ebenes Blech, unterbunden. Üblicherweise teilt dabei die Trennwand den Kolonneninnenraum in einen Zuführteil, einen Entnahmeteil, einen oberen gemeinsamen Kolonnenbereich sowie einen unteren gemeinsamen Kolonnenbereich. Zwischen dem Zuführbereich und dem Entnahmebereich ist die Trennwand angeordnet, die in diesen Kolonnenbereichen eine Quervermischung von Flüssigkeits- und Brüdenströmen über den gesamten Kolonnenquerschnitt unterbindet. Dadurch ist es möglich, am Seitenabzug ein Produkt in reiner Form zu gewinnen. Die Trennwand kann fest verschweißt oder auch nur lose gesteckt sein, wobei die letztere Variante den Vorteil niedrigerer Investitionskosten aufweist.

Für die Durchführung der destillativen Auftrennung des Reaktionsgemisches im vorliegenden Verfahren in einer Trennwandkolonne wird das nahezu wasserfreie Reaktionsgemisch einer Trennwandkolonne mit flüssigem Seitenabzug zugeführt, die bevorzugt 40 bis 100 theoretische Trennstufen, insbesondere 50 bis 90, besonders bevorzugt 60 bis 85 theoretische Trennstufen aufweist, und die bei einem Kopfdruck von 5 bis 500 mbar, bevorzugt von 10 bis 300 mbar, besonders bevorzugt von 15 bis 100 mbar, betrieben wird. In der Trennwandkolonne wird das Reaktionsgemisch kontinuierlich in drei Fraktionen destillativ aufgetrennt: in eine Leichtsiederfraktion, die nicht umgesetzte Edukte enthält und die vorzugsweise in die Reaktionsstufe recycliert wird, in eine Mittelsiederfraktion, die das reine Dialdehyd-Monoacetal enthält, d.h. ein Gemisch mit einem Gehalt an Wertprodukt von mindestens 98 Gew.-%, vorzugsweise mindestens 99 Gew.-% sowie eine Hochsiederfraktion, die das Diacetal sowie höhersiedende Komponenten enthält.

Als Sumpfverdampfer für die Trennwandkolonne sind Fallfilmverdampfer besonders geeignet, da sie eine schonende Verdampfung gewährleisten und das thermisch empfindliche Produkt nicht belasten.

Bezüglich der trennwirksamen Einbauten gibt es grundsätzlich keine Einschränkung, d.h. es können Böden, Füllkörper oder Packungen, beispielsweise Blech- oder Gewebepackungen, vorzugsweise mit spezifischen Oberflächen von 250 bis 750 m²/m³, eingesetzt werden. Gewebepackungen sind aufgrund ihres geringeren Druckverlustes pro Trennstufe sowie ihrer besseren Trennleistung bei Vakuumdestillationen, wie sie vorliegend eingesetzt werden, besonders bevorzugt.

Der Sumpfaustrag der Trennwandkolonne wird anschließend bevorzugt einem Dünnschichtverdampfer zugeführt und darin, vorzugsweise bei einem Druck von etwa 10 mbar in zwei Ströme aufgeteilt: das flüchtige Diacetal wird dampfförmig abgetrennt, kondensiert und zur Spaltung in die Acetalisierungsstufe recycliert. Die schwersiedenden Polymere werden thermisch verwertet.

Die Trennwandkolonne ist bevorzugt dergestalt aufgeteilt, dass sämtliche Kolonnenbereiche, d.h. der obere gemeinsame Kolonnenbereich, der Verstärkungsteil des Zuführbereichs, der Abtriebsteil des Zuführbereichs, der Verstärkungsteil des Entnahmebereichs, der Abtriebsteil des Entnahmebereichs sowie der untere gemeinsame Kolonnenbereich jeweils 5 bis 50%, bevorzugt 15 bis 30% der Gesamtzahl der theoretischen Trennstufen der Trennwandkolonne aufweisen.

Weiter bevorzugt ist die Trennwandkolonne dergestalt ausgebildet, dass die Summe der Anzahl der theoretischen Trennstufen der beiden Teilbereiche des Zuführbereichs, d.h. des Verstärkungsteils und des Abtriebsteils desselben 80 bis 110%, bevorzugt 90 bis 100% der Summe der Anzahl der theoretischen Trennstufen der beiden Teilbereiche (Verstärkungsteil sowie Abtriebsteil) des Entnahmebereichs, beträgt.

Zulauf und Seitenabzug können auf unterschiedlicher Höhe in der Trennwandkolonne angeordnet sein, wobei der Zulauf bevorzugt um 1 bis 8, besonders bevorzugt um 3 bis 5 theoretische Trennstufen höher oder niedriger angeordnet ist als der Seitenabzug.

Das Aufteilungsverhältnis der Flüssigkeit am oberen Ende der Trennwand wird vorzugsweise so eingestellt, dass die Konzentration an denjenigen Komponenten der Hochsiederfraktion, für die im Seitenabzug ein bestimmter Grenzwert vorgegeben ist, in der Flüssigkeit am oberen Ende der Trennwand 10 bis 80%, bevorzugt 30 bis 50% des Wertes beträgt, der für das Seitenabzugsprodukt vorgegeben ist. Bei einem höheren Gehalt an Hochsiedern wird die Flüssigkeitsaufteilung so eingestellt, dass mehr Flüssigkeit auf den Zuführbereich geleitet wird, wogegen bei einer niedrigeren Konzentration an Hochsiedern weniger Flüssigkeit auf den Zuführbereich geleitet wird.

Die Heizleistung im Sumpfverdampfer wird vorzugsweise so eingestellt, dass die Konzentration an denjenigen der Komponenten der Leichtsiederfraktion, für die im Seitenabzug ein bestimmter Grenzwert vorgegeben ist, am unteren Ende der Trennwand so eingestellt wird, dass sie 10 bis 80%, bevorzugt 30 bis 50% des Wertes beträgt, der für das Seitenabzugsprodukt vorgegeben ist. Bei einem höheren Gehalt an Komponenten der Leichtsiederfraktion wird die Heizleistung erhöht und bei einem niedrigeren Gehalt derselben verringert.

Die Destillatentnahme erfolgt temperaturgeregelt, wobei als Regeltemperatur eine Meßstelle im oberen gemeinsamen Kolonnenbereich verwendet wird, die um 3 bis 8, bevorzugt um 4 bis 6 theoretische Trennstufen unterhalb des oberen Endes der Kolonne angeordnet ist.

Die Entnahme des Sumpfproduktes erfolgt ebenfalls temperaturgeregelt. Als Regeltemperatur gilt eine Meßstelle im unteren gemeinsamen Kolonnenbereich, die um 3 bis 8, bevorzugt um 4 bis 6 theoretische Trennstufen oberhalb des unteren Endes der Kolonne angeordnet ist.

Die Entnahme des Seitenprodukts erfolgt bevorzugt standgeregelt, wobei als Regelgröße der Flüssigkeitsstand im Sumpfverdampfer dient.

Ein Kostenvergleich zwischen den beiden Varianten der destillativen Auftrennung in zwei hintereinander geschalteten Destillationskolonnen einerseits und in eine Trennwandkolonne andererseits zeigt, dass die Trennwandkolonne sowohl bezüglich der Investitionskosten als auch der Energiekosten um etwa 30% günstiger ist. Ein weiterer Vorteil der Auftrennung in einer Trennwandkolonne ist die deutliche Verringerung der thermischen Belastung des empfindlichen Produktes, die aus der Verkürzung der Verweilzeit im Sumpfverdampfer, insbesondere aufgrund der Reduzierung auf einen einzigen Sumpfverdampfer, resultiert.

In einer besonders vorteilhaften Verfahrensvariante wird das weitgehend wasserfreie Reaktionsgemisch vor der Zuführung desselben zur destillativen Auftrennung bei 80 bis 130°C getempert.

Es wurde überraschend gefunden, dass sich die Viskosität des Reaktionsgemisches durch Tempern signifikant reduzieren lässt, insbesondere in einen Bereich, in dem dasselbe über Pumpen einfach transportierbar wird. Darüber hinaus wird durch das Tempern ein signifikanter Anstieg an Wertprodukt, dem aliphatischen Dialdehyd-Monoacetal, im Reaktionsgemisch erreicht.

Das Tempern erfolgt erfindungsgemäß bei Temperaturen im Bereich von 80 bis 130°C, bevorzugt von 90 bis 110°C. Die Temperdauer ist unkritisch: eine Mindestdauer von 15 Minuten kann ausreichend sein, eine Obergrenze ist für den erfindungsgemäßen Erfolg nicht ausschlaggebend, sondern allenfalls aufgrund wirtschaftlicher Überlegungen denkbar. Bevorzugt wird 30 Minuten bis 4 Stunden lang, besonders bevorzugt 1 Stunde lang getempert.

Die Druckbedingungen beim Tempern sind nicht kritisch:

Getempert werden kann im Vakuum, bei Überdruck oder bei Atmosphärendruck, bevorzugt jedoch bei Atmosphärendruck.

In einer weiteren besonders vorteilhaften Verfahrensvariante führt man die destillative Auftrennung des gegebenenfalls getemperten Reaktionsgemisches unter Zugabe eines hochsiedenden Verdünnungsmittels in den unteren Bereich der ersten Destillationskolonne oder in den unteren gemeinsamen Kolonnenbereich der Trennwandkolonne durch.

Das hochsiedende Verdünnungsmittel muss mit dem Reaktionsgemisch mischbar sein, er darf mit demselben nicht reagieren und soll einen niedrigeren Dampfdruck aufweisen als jede Einzelkomponente des Reaktionsgemisches sowie als das Reaktionsgemisch. Vorzugsweise setzt man das hochsiedende Verdünnungsmittel in einem Anteil von 1 bis 30 Gew.-%, bevorzugt von 2 bis 20 Gew.-%, besonders bevorzugt von 5 bis 15 Gew.-%, bezogen auf das destillativ aufzutrennende Gemisch, zu.

Besonders geeignet ist eine Substanz oder ein Gemisch von Substanzen, ausgewählt aus den nachstehend aufgeführten Gruppen: Alkane, Aromate oder Polyether, bevorzugt Polypropylenglykole oder Polyethylenglykole, besonders bevorzugt Polyethylenglykol mit der mittleren Molekularmasse von 300.

Durch die Zugabe des hochsiedenden Verdünnungsmittels wird ein Verbacken und Durchpolymerisieren des Destillationssumpfes an den Wärmetauscherflächen verhindert, und somit die Ausbeute an Wertprodukt verbessert.

Die Erfindung wird im Folgenden anhand von Ausführungsbeispielen sowie einer Zeichnung näher erläutert.

### Beispiele 1 bis 3: Umsetzung von Glutaraldehyd mit Ethylenglykol zum 2-(3-Formylpropyl)-1,3-dioxolan (FPDO).

### Beispiel 1: gleichzeitiges Abdestillieren des Lösungswassers und Zugabe von Ethylenglykol

In einer 1-1-Rührapparatur mit aufgesetzter 10-cm-Füllkörperkolonne mit Raschig-Ringen und Destillationsbrücke wurden 800 g einer Glutaraldehyd-Lösung (50%ig in Wasser) vorgelegt. Bei 60 bis 65°C Innentemperatur und einem Vakuum von 200 mbar wurde das Wasser abdestilliert. Sobald das erste Destillat erhalten wurde, wurde gleichzeitig zum Abdestillieren des Wassers innerhalb von zwei Stunden eine Lösung von 1,2 g Orthophosphorsäure (99%ig) in 248 g Ethylenglykol zugetropft. Der Reaktionsansatz wurde nach der Zugabe bei 65°C/200 mbar noch eine Stunde nachgerührt. Danach wurde das Vakuum schrittweise auf 25 mbar verbessert, die Innentemperatur auf 85°C erhöht und dabei alles Wasser abdestilliert. Es wurden 530 g einer sehr viskosen, farblosen Rohlösung erhalten. Zusammensetzung (GC Flächen-%): 50,5% FPDO, 36,9% 1,3-Bis(1,3-dioxolan-2-yl)-propan (Bisacetal), 9,0% Glutaraldehyd.

### Beispiel 2: Abdestillieren des Lösungswassers gefolgt von Zugabe des Ethylenglykols

In einer 2-1-Rührapparatur mit aufgesetzter 10-cm-Füllkörperkolonne mit Raschig-Ringen und Destillationsbrücke wurden 1200 g einer Glutaraldehyd-Lösung (50%ig in Wasser) vorgelegt und danach bei einer Innentemperatur von 70 bis 80°C und einem Vakuum von 150 mbar das Wasser abdestilliert. Bei einer Innentemperatur von 75 bis 83°C wurde anschließend innerhalb von 90 Minuten eine Lösung von 2 g Orthophosphorsäure (99%ig) in 372 g Ethylenglykol zugetropft und der Reaktionsansatz anschließend 90 Minuten nachgerührt. Danach wurde unter Anlegen von Vakuum, das schrittweise von 100 auf 50 mbar verbessert wurde, das Reaktionswasser bei einer Innentemperatur von 70 bis 88°C abdestilliert. Es wurden 850 g einer sehr viskosen, farblosen Rohlösung erhalten. Zusammensetzung (GC Flächen-%): 50,7% FPDO, 25,0% 1,3-Bis(1,3-dioxolan-2-yl)-propan (Bisacetal), 12,7% Glutaraldehyd, 3,0% Ethylenglykol.

### Beispiel 3: Abdestillieren von Lösungs- und Reaktionswasser

In einer 1-1-Rührapparatur mit aufgesetzter 10-cm-Füllkörperkolonne mit Raschig-Ringen und Destillationsbrücke wurden 800 g einer Glutaraldehyd-Lösung (50%ig in Wasser), 248 g Ethylenglykol und 1,2 g Orthophosphorsäure (99%ig) vorgelegt. Die Reaktionsmischung wurde bei 60°C 45 Minuten gerührt. Danach wurde bei 180 mbar innerhalb von 3 Stunden Wasser abdestilliert. Anschließend wurde das Vakuum schrittweise auf 30 mbar verbessert und die Innentemperatur auf 90°C erhöht, um alles Wasser abzudestillieren. Es wurden 568 g einer sehr viskosen, leicht trüben Rohlösung erhalten. Zusammensetzung (GC Flächen-%): 56,4% FPDO, 19,5% 1,3-Bis(1,3-dioxolan-2-yl)-propan (Bisacetal), 14,8% Glutaraldehyd, 2,2% Ethylenglykol.

### Vergleichsbeispiel Tempern

Eine nach Beispiel 1 hergestellte Rohlösung mit einem gaschromatographisch, mit internem Standard bestimmtem Gehalt an 50 Gew.-% FPDO wurde bei Normaldruck bei 60°C unter Schutzgas getempert. Der FPDO-Gehalt sank nach 24-stündigem Tempern auf 36 Gew.-% FPDO und nach 72-stündigem Tempern auf 29,4 Gew.-% FPDO.

### Beispiel 4: Tempern

Verschiedene Proben einer nach Beispiel 1 hergestellten Rohlösung, die kurze Zeit bei 60°C gelagert worden war, und deren gaschromatographisch mit internem Standard bestimmter Gehalt an FPDO 35,3 Gew.-% betrug, wurden unter Variation von Temperatur und Zeit getempert. Für die getemperten Lösungen wurde der Gehalt an Wertprodukt FPDO sowie die kinematischen Viskositäten nach DIN 51562 bestimmt.

Die Ergebnisse sind in der nachstehenden Tabelle 1 aufgeführt:

| | Temperatur | Zeit [h] | Gew.-% FPDO |
|---|---|---|---|
| 4.0 | | 0 | 35,3 |
| 4.1 | 90°C | 1 | 40,0 |
| 4.2 | | 3 | 41,4 |
| 4.3 | | 5 | 41,7 |
| 4.4 | 100°C | 1 | 43,4 |
| 4.5 | | 3 | 43,2 |
| 4.6 | | 5 | 41,7 |
| 4.7 | 110°C | 1,5 | 45,6 |
| 4.8 | | 3 | 46,6 |
| 4.9 | 120°C | 1,5 | 47,5 |
| 4.10 | | 3 | 46,0 |

Die kinematische Viskosität der Rohlösung nach dem Vergleichsbeispiel 4.0, d.h. der ungetemperten Probe, betrug 6040 mm²/s bei 20°C, dagegen betrug die Viskosität der Probe 4.8 (3 Stunden lang bei 110°C getempert) nur noch 17,5 mm²/s bei 20°C. Das Tempern führt somit zu einer signifikanten Reduzierung der Viskosität. Darüber hinaus steigt der Gehalt an Wertprodukt FPDO deutlich an, wie in der letzten Spalte der obenstehenden Tabelle erkennbar.

### Vergleichsbeispiel Destillation

750 g einer nach Beispiel 2 hergestellten Rohlösung mit einem Gehalt von 50 Gew.-% FPDO wurden diskontinuierlich in einer 60 cm-Füllkörperkolonne mit Raschig-Ringen bei einer Sumpftemperatur von 150°C, einem Vakuum von 1,5 mbar und einer Verweilzeit im Sumpf von etwa 10 Stunden, destilliert. Insgesamt konnten destillativ lediglich 230 g FPDO abgetrennt werden, was einer Destillationsausbeute von nur 60% entspricht. Der Sumpf war extrem zähflüssig und entstandener Feststoff hat den unteren Kolonnenbereich zupolymerisiert.

### Beispiel 5: Simulation der thermischen Belastung des Gemisches in einer Kolonne ohne Zusatz von hochsiedenden Verdünnungsmittel

Zur Simulation der thermischen Belastung der Rohlösung bei der Destillation in einer Kolonne mit Fallfilm-Umlaufverdampfer wurden 500 g einer nach Beispiel 1 hergestellten und 2 Stunden bei 95°C getemperten Rohlösung kontinuierlich mit einer Zulaufgeschwindigkeit von 500 g/h und einer Verweilzeit von ca. 1 Minute bei einer Temperatur von 150 bis 155°C und einem Vakuum von 2 mbar destilliert. Nach der Hälfte des Zulaufes waren starke schwarze Ablagerungen zu beobachten und der Sumpfablauf polymerisierte zu, so dass der Versuch abgebrochen werden musste.

### Beispiel 6: Simulation der thermischen Belastung des Gemisches in einer Kolonne mit Zusatz von hochsiedenden Verdünnungsmitteln

Zur Simulation der thermischen Belastung der Rohlösung bei der Destillation in einer Kolonne mit Fallfilm-Umlaufverdampfer wurden 3700 g einer nach Beispiel 1 hergestellten und 2 Stunden bei 95°C getemperten Rohware, jedoch in Abmischung mit 10 Gew.-% Polyethylenglykol der Molmasse 300, kontinuierlich an einem Dünnschichtverdampfer bei einer Temperatur von 135°C und einem Vakuum von 1 mbar destilliert. Die Zulaufgeschwindigkeit betrug, wie in Beispiel 5, 500 g/h und die Verweilzeit etwa 1 Minute. Der Versuch wurde nach 7 Stunden abgebrochen, ohne dass ein Belag auf der Apparatur zu beobachten gewesen wäre. Es wurden 996 g Destillat und 1670 g Sumpfaustrag erhalten.

### Beispiel 7: Destillation ohne Zusatz von hochsiedenden Verdünnungsmitteln

In einer Kolonne (Durchmesser 300 mm, Sulzer Packung, 60 theoretische Trennstufen) wurden in zwei Stufen 10 t einer analog zu Beispiel 1 hergestellten Rohware mit einem Gehalt an FPDO von 40 Gew.-% kontinuierlich destilliert. Zunächst fiel bei der ersten Stufe (20 mbar; Verweilzeit: ca. 4 h) im flüssigen Seitenabzug das Monoacetal, FPDO mit einer Reinheit von ca. 95% an. In einer zweiten Stufe (15 mbar) fiel das Reinprodukt FPDO dann ebenfalls im dampfförmigen Seitenabzug an. Jeweils über Kopf erhielt man nicht umgesetzten Glutaraldehyd und Ethylenglykol; über den Sumpf der ersten Destillationsstufe erhielt man das Bisacetal, das wieder in die Synthese zurückgeführt wurde. Es wurden 3,8 t FPDO mit einer Reinheit von > 99% erhalten. Die Destillationsausbeute betrug 95%.

Es zeigen im Einzelnen:

| | |
|---|---|
| Figur 1 | ein Destillationsschema mit zwei hintereinander angeordneten Destillationskolonnen, |
| Figur 2 | ein Destillationsschema mit einer Trennwandkolonne und |
| Figur 3 | ein Destillationsschema mit einer Trennwandkolonne unter Angabe der Regeleinrichtungen. |

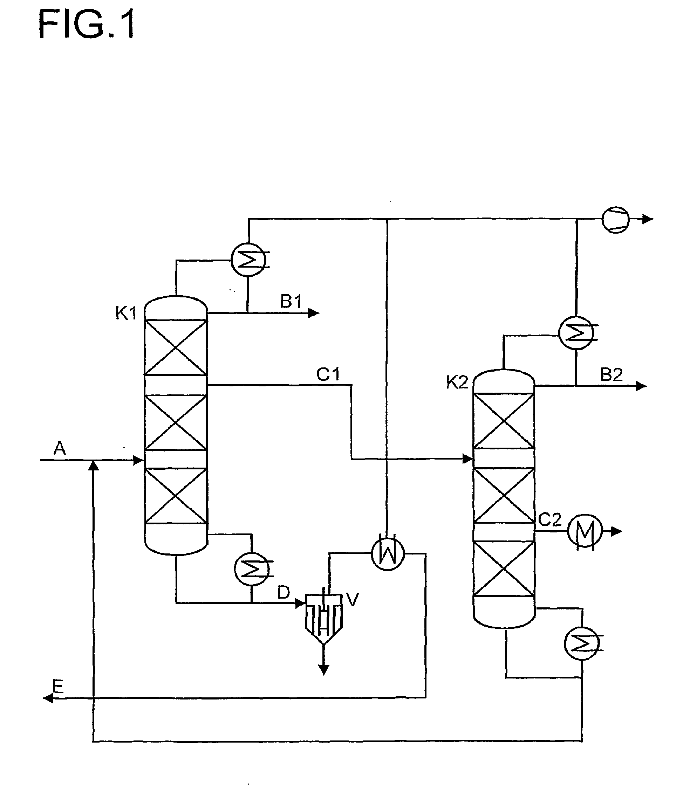

Figur 1 zeigt eine erste Destillationskolonne K1, der das wasserfreie Reaktionsgemisch (Strom A) im mittleren Bereich zugeführt wird. Die Destillationskolonne K1 ist mit einem Sumpfverdampfer sowie einem Kondensator am Kolonnenkopf ausgestattet. Der Kopfstrom wird im Kondensator am Kolonnenkopf kondensiert, teilweise als Strom B1, der überwiegend Glutardialdehyd enthält, abgezogen und im übrigen als Rücklauf erneut auf die Kolonne aufgegeben. Aus dem Verstärkungsteil der Kolonne wird als flüssiger Seitenabzug Roh-FPDO (Strom C1) abgezogen. Der Sumpfstrom D wird in einem Dünnschichtverdampfer V in zwei Ströme aufgeteilt, und zwar einen Kopfstrom, enthaltend das flüchtige Diacetal, das teilweise als Strom E der Synthese recycliert wird sowie einen Sumpfstrom, umfassend Hochsieder, der ausgeschleust wird.

Das Roh-FPDO (Strom C1) wird der zweiten Destillationskolonne K2 im mittleren Bereich derselben aufgegeben. Die Kolonne K2 ist ebenfalls mit einem Kondensator am Kolonnenkopf sowie mit einem Sumpfverdampfer ausgestattet. Der Kopfstrom der Kolonne K2 wird im Kondensator am Kolonnenkopf kondensiert, teilweise als Strom B2, der überwiegend aus Glutardialdehyd besteht, abgezogen und im übrigen als Rücklauf erneut auf die Kolonne aufgegeben. Aus dem Abtriebsteil der Kolonne K2 wird ein Rein-FPDO enthaltender Strom (Strom C2) dampfförmig abgezogen und kondensiert. Der Sumpfstrom wird in die Kolonne K1 recycliert.

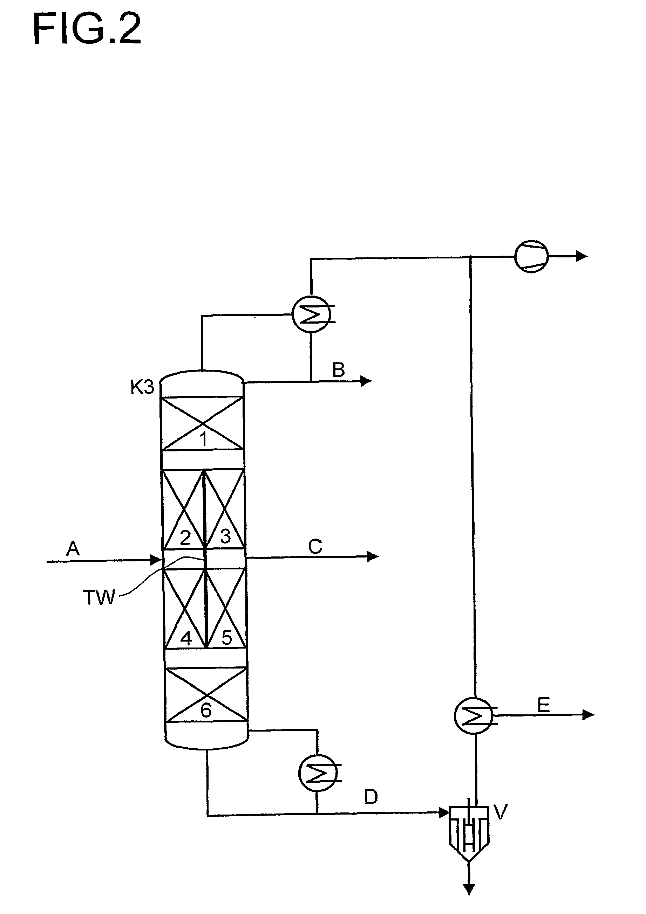

Figur 2 zeigt eine Trennwandkolonne K3 mit in Kolonnenlängsrichtung angeordneter Trennwand TW, die den Kolonneninnenraum in einen Zuführbereich mit Verstärkungsteil 2 und Abtriebsteil 4 sowie in einen Entnahmebereich mit Verstärkungsteil 3 und Abtriebsteil 5 auftrennt, sowie in einen oberen gemeinsamen Kolonnenbereich 1 und einen unteren gemeinsamen Kolonnenbereich 6. Der Trennwandkolonne wird das wasserfreie Reaktionsgemisch als Strom A in den mittleren Bereich des Zuführbereichs zugeführt, der Kopfstrom wird in einem Kondensator am Kolonnenkopf kondensiert, teilweise als Strom B, umfassend überwiegend Glutardialdehyd, abgezogen und im übrigen als Rücklaufstrom erneut auf die Kolonne aufgegeben. Aus dem Entnahmebereich der Kolonne wird FPDO (Strom C) abgezogen. Der Sumpfstrom D wird in einem Dünnschichtverdampfer V in einen Kopfstrom, umfassend überwiegend das Diacetal aufgetrennt, der als Strom I in die Synthese recycliert wird sowie in einen Sumpfstrom, der ausgeschleust wird.

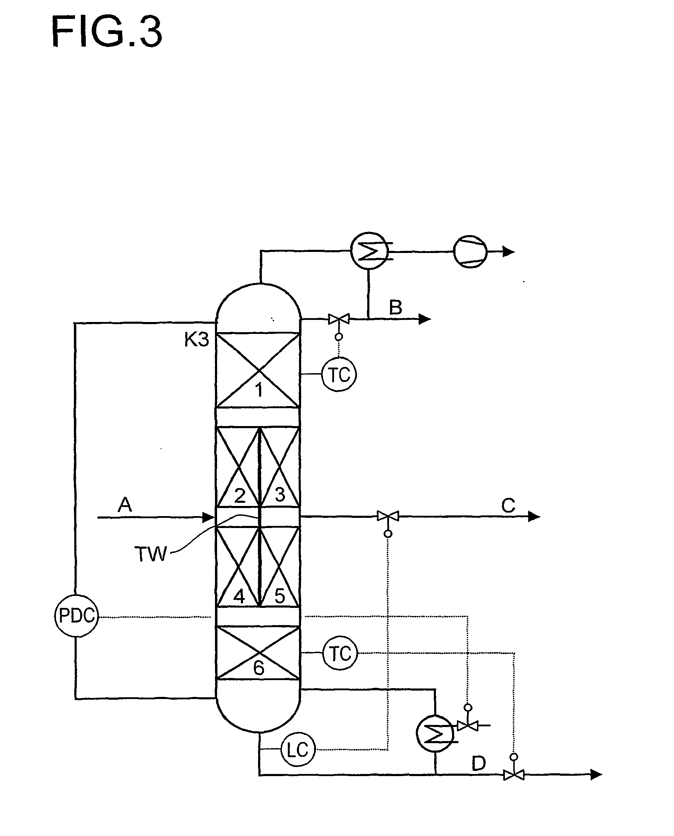

Die schematische Darstellung in Figur 3 verdeutlicht die Regeleinrichtungen für die Trennwandkolonne K3. Mit TC sind Temperaturregler bezeichnet, LC ist ein Flüssigkeitsstand-Regler und PDC ist ein Differenzdruck-Messer.

## Patentansprüche

1. Verfahren zur Gewinnung eines reinen aliphatischen Dialdehyd-Monoacetals, das zu mindestens 98 Gew.-% aus dem Wertprodukt, aliphatisches Dialdehyd-Monoacetat, gebildet ist, durch einstufige Umsetzung des entsprechenden aliphatischen Dialdehyds oder eines Vorläufers des entsprechenden aliphatischen Dialdehyds mit einem oder mehreren aliphatischen ein- oder mehrwertigen Alkoholen, wobei man Wasser destillativ abtrennt, unter Erhalt eines Reaktionsgemisches, das man destillativ auftrennt, **dadurch gekennzeichnet, dass** man die destillative Auftrennung kontinuierlich durchführt, in einer Trennwandkolonne, wobei man reines aliphatisches Dialdehyd-Monoacetal als Seitenabzug der Trennwandkolonne erhält oder in zwei Destillationskolonnen, wobei man in der ersten Destillationskolonne rohes aliphatisches Dialdehyd-Monoacetal als Seitenabzug erhält, das rohe aliphatische Dialdehyd-Monoacetal der zweiten Destillationskolonne zuführt und aus der zweiten Destillationskolonne reines aliphatisches Dialdehyd-Monoacetal als Seitenabzug erhält.

2. Verfahren nach Anspruch 1, **dadurch gekennzeichnet, dass** man das Reaktionsgemisch vor der destillativen Auftrennung bei 80 bis 130°C tempert.

3. Verfahren nach einem der Ansprüche 1 oder 2, **dadurch gekennzeichnet, dass** man das Reaktionsgemisch mindestens 15 Minuten lang, bevorzugt 30 Minuten bis 4 Stunden lang, besonders bevorzugt 1 Stunde lang, bevorzugt bei 90 bis 110°C tempert.

4. Verfahren nach einem der Ansprüche 1 bis 3, **dadurch gekennzeichnet, dass** der aliphatische Dialdehyd eine Substanz aus der nachfolgenden Aufzählung ist: Malonaldehyd, Succinaldehyd, Glutaraldehyd oder Adipinaldehyd.

5. Verfahren nach Anspruch 4, **dadurch gekennzeichnet, dass** man als aliphatischen Dialdehyd Glutaraldehyd, bevorzugt in wässriger Lösung, besonders bevorzugt als 50 gew.-%ige wässrige Lösung einsetzt, oder dessen Vorläufer, 2-Hydroxy-3,4-dihydo-2H-pyran.

6. Verfahren nach einem der Ansprüche 1 bis 5, **dadurch gekennzeichnet, dass** der aliphatische ein- oder mehrwertige Alkohol ein Diol ist, insbesondere Ethylenglykol, 1,2-Propylenglykol, 1,3-Propylenglykol, 1,2-Butandiol, 1,3-Butandiol oder 1,4-Butandiol, besonders bevorzugt Ethylenglykol.

7. Verfahren nach Anspruch 5 oder 6, **dadurch gekennzeichnet, dass** man Glutaraldehyd mit Ethylenglykol in einem Molverhältnis im Bereich von 1:1,5 bis 1,5:1, bevorzugt von 1:1,2 bis 1,2:1, besonders bevorzugt von 1,0:1,0, umsetzt.

8. Verfahren nach einem der Ansprüche 1 bis 7, **dadurch gekennzeichnet, dass** man die Umsetzung in Gegenwart eines sauren Katalysators durchführt, insbesondere eines Kationenaustauschers, einer Mineralsäure, bevorzugt Schwefelsäure, Chlorwasserstoffsäure, besonders bevorzugt Orthophosphorsäure oder einer organischen Säure, insbesondere Essigsäure, p-Toluolsulfonsäure oder Methansulfonsäure, in einer Konzentration von 0,02 bis 5 Gew.-%, bevorzugt von 0,1 bis 1 Gew.-%, besonders bevorzugt von 0,3 Gew.-%, bezogen auf das Gesamtgewicht der Reaktionsmischung.

9. Verfahren nach einem der Ansprüche 1 bis 8, **dadurch gekennzeichnet, dass** man das gegebenenfalls getemperte Reaktionsgemisch kontinuierlich in zwei Destillationskolonnen auftrennt, wobei man in einer ersten Destillationskolonne das rohe aliphatische Dialdehyd-Monoacetal als Seitenabzug und in einer zweiten Destillationskolonne das reine aliphatische Dialdehyd-Monoacetal als Seitenabzug abtrennt.

10. Verfahren nach einem der Ansprüche 1 bis 8, **dadurch gekennzeichnet, dass** man das gegebenenfalls getemperte Reaktionsgemisch in einer Trennwandkolonne auftrennt mit einer vertikal, in Kolonnenlängsrichtung angeordneten Trennwand, die die Kolonne in einen Zuführbereich, einen Entnahmebereich, einen unteren gemeinsamen Kolonnenbereich sowie einen oberen gemeinsamen Kolonnenbereich aufteilt, unter Gewinnung von reinem aliphatischem Dialdehyd-Monoacetal als Seitenabzug aus dem Entnahmebereich.

11. Verfahren nach einem der Ansprüche 1 bis 10, **dadurch gekennzeichnet, dass** man die destillative Auftrennung des gegebenenfalls getemperten Reaktionsgemisches unter Zugabe eines hochsiedenden Verdünnungsmittels in den unteren Bereich der ersten Destillationskolonne oder in den unteren gemeinsamen Kolonnenbereich der Trennwandkolonne durchführt.

12. Verfahren nach Anspruch 11, **dadurch gekennzeichnet, dass** das hochsiedende Verdünnungsmittel eine Substanz oder ein Gemisch von Substanzen, ausgewählt aus den nachstehend aufgeführten Gruppen ist: Alkane, Aromate oder Polyether, bevorzugt Polypropylenglykole oder Polyethylenglykole, besonders bevorzugt Polyethylen glykol mit der mittleren Molekularmasse von 300.

## Claims

1. A process for obtaining a pure aliphatic dialdehyde monoacetal which is formed of at least 98% by weight of the product of value, aliphatic dialdehyde monoacetal, by single-stage reaction of the corresponding aliphatic dialdehyde or a precursor of the corresponding aliphatic dialdehyde with one or more aliphatic mono- or polyhydric alcohols while distillatively removing water to obtain a reaction mixture which is separated distillatively, which comprises carrying out the distillative separation continuously in a dividing wall column to obtain pure aliphatic dialdehyde monoacetal as a sidestream from the dividing wall column, or in two distillation columns to obtain crude aliphatic dialdehyde monoacetal as a sidestream in the first distillation column, feed the crude aliphatic dialdehyde monoacetal to the second distillation column and obtain pure aliphatic dialdehyde monoacetal as the sidestream from the second distillation column.

2. The process according to claim 1, wherein the reaction mixture is heated to from 80 to 130°C before the distillative separation.

3. The process according to either of claims 1 or 2, wherein the reaction mixture is heated for at least 15 minutes, preferably from 30 minutes to 4 hours, more preferably for 1 hour, preferably at from 90 to 110°C.

4. The process according to any of claims 1 to 3, wherein the aliphatic dialdehyde is a substance from the following list: malonaldehyde, succinaldehyde, glutaraldehyde or adipaldehyde.

5. The process according to claim 4, wherein the aliphatic dialdehyde used is glutaraldehyde, preferably in aqueous solution, more preferably as a 50% by weight aqueous solution, or its precursor 2-hydroxy-3,4-dihydro-2H-pyran.

6. The process according to any of claims 1 to 5, wherein the aliphatic mono- or polyhydric alcohol is a diol, in particular ethylene glycol, 1,2-propylene glycol, 1,3-propylene glycol, 1,2-butanediol, 1,3-butanediol or 1,4-butanediol, more preferably ethylene glycol.

7. The process according to claim 5 or 6, wherein glutaraldehyde is reacted with ethylene glycol in a molar ratio in the range from 1:1.5 to 1.5:1, preferably from 1:1.2 to 1.2:1, more preferably of 1.0:1.0.

8. The process according to any of claims 1 to 7, wherein the reaction is carried out in the presence of an acidic catalyst, in particular of a cation exchanger, of a mineral acid, preferably sulfuric acid, hydrochloric acid, more preferably orthophosphoric acid, or an organic acid, in particular acetic acid, p-toluenesulfonic acid or methanesulfonic acid, in a concentration of from 0.02 to 5% by weight, preferably from 0.1 to 1% by weight, more preferably of 0.3% by weight, based on the total weight of the reaction mixture.

9. The process according to any of claims 1 to 8, wherein the optionally heated reaction mixture is continuously separated in two distillation columns to remove the crude aliphatic dialdehyde monoacetal as a sidestream in a first distillation column and the pure aliphatic dialdehyde monoacetal as a sidestream in a second distillation column.

10. The process according to any of claims 1 to 9, wherein the optionally heated reaction mixture is separated in a dividing wall column having a vertical dividing wall which is disposed in the longitudinal direction of the column and divides the column into a feed region, a takeoff region, a lower combined column region and also an upper combined column region, to recover pure aliphatic dialdehyde monoacetal as a sidestream from the withdrawal region.

11. The process according to any of claims 1 to 10, wherein the distillative separation of the optionally heated reaction mixture is carried out with the addition of a high-boiling diluent in the lower region of the first distillation column or in the upper combined column region of the dividing wall column.

12. The process according to claim 11, wherein the high-boiling diluent is a substance or a mixture of substances selected from the following listed groups: alkanes, aromatics or polyethers, preferably polypropylene glycols or polyethylene glycols, more preferably polyethylene glycol having an average molecular mass of 300.

## Revendications

1. Procédé d'obtention d'un monoacétal de dialdéhyde aliphatique pur formé pour au moins 98 % en poids à partir du produit de valeur monoacétal de dialdéhyde aliphatique, par réaction en une étape du dialdéhyde aliphatique correspondant ou d'un précurseur du dialdéhyde aliphatique correspondant avec un ou plusieurs alcools monovalents ou plurivalents aliphatiques, de l'eau étant isolée par distillation, avec obtention d'un mélange réactionnel qui est séparé par distillation, **caractérisé en ce que** la séparation par distillation est mise en oeuvre en continu, dans une colonne à paroi de séparation, un monoacétal de dialdéhyde aliphatique pur étant obtenu en tant qu'évacuation latérale de la colonne à paroi de séparation ou dans deux colonnes de distillation, un monoacétal de dialdéhyde aliphatique brut étant obtenu en tant qu'évacuation latérale dans la première colonne de distillation, le monoacétal de dialdéhyde aliphatique brut alimentant la deuxième colonne de distillation et un monoacétal de dialdéhyde aliphatique pur étant obtenu en tant qu'évacuation latérale à partir de la seconde colonne de distillation.

2. Procédé selon la revendication 1, **caractérisé en ce que** le mélange réactionnel est recuit à une température de 80 à 130 °C avant la séparation par distillation.

3. Procédé selon la revendication 1 ou 2, **caractérisé en ce que** le mélange réactionnel est recuit pendant au moins 15 minutes, de préférence de 30 minutes à 4 heures, mieux encore pendant une 1 heure, de préférence à une température de 90 à 110 °C.

4. Procédé selon l'une quelconque des revendications 1 à 3, **caractérisé en ce que** le dialdéhyde aliphatique est une substance comprise dans l'énumération suivante : malonaldéhyde, succinaldéhyde, glutaraldéhyde ou adipinaldéhyde.

5. Procédé selon la revendication 4, **caractérisé en ce que** du glutaraldéhyde est utilisé en tant que dialdéhyde aliphatique, de préférence dans une solution aqueuse, particulièrement préférablement en tant que solution aqueuse à 50 % en poids, ou son précurseur, du 2-hydroxy-3,4-dihydro-2H-pyrane.

6. Procédé selon l'une quelconque des revendications 1 à 5, **caractérisé en ce que** l'alcool monovalent ou plurivalent aliphatique est un diol, en particulier de l'éthylèneglycol, du 1,2-propylèneglycol, du 1,3-propylèneglycol, du 1,2-butanediol, du 1,3-butanediol ou du 1,4-butanediol, particulièrement préférablement de l'éthylèneglycol.

7. Procédé selon la revendication 5 ou 6, **caractérisé en ce que** du glutaraldéhyde est amené à réagir avec de l'éthylèneglycol en un rapport molaire dans la plage de 1:1,5 à 1,5:1, de préférence de 1:1,2 à 1,2:1, mieux encore de 1,0:1,0.

8. Procédé selon l'une quelconque des revendications 1 à 7, **caractérisé en ce que** la réaction est mise en oeuvre en présence d'un catalyseur acide, en particulier d'un échangeur de cations, d'un acide minéral, de préférence de l'acide sulfurique, de l'acide chlorhydrique, mieux encore de l'acide orthophosphorique ou d'un acide organique, en particulier de l'acide acétique, de l'acide p-toluènesulfonique ou de l'acide méthanesulfonique, en une concentration de 0,02 à 5 % en poids, de préférence de 0,1 à 1 % en poids, mieux encore de 0,3 % en poids, par rapport au poids total du mélange réactionnel.

9. Procédé selon l'une quelconque des revendications 1 à 8, **caractérisé en ce que** le mélange réactionnel éventuellement recuit est séparé en continu dans deux colonnes de distillation, le monoacétal de dialdéhyde aliphatique brut étant isolé en tant qu'évacuation latérale dans une première colonne de distillation et le monoacétal de dialdéhyde aliphatique pur en tant qu'évacuation latérale dans une deuxième colonne de distillation.

10. Procédé selon l'une quelconque des revendications 1 à 9, **caractérisé en ce que** le mélange réactionnel éventuellement recuit est séparé dans une colonne à paroi de séparation, avec une cloison séparatrice agencée de manière verticale dans le sens longitudinal de la colonne, qui divise la colonne en un secteur d'alimentation, un secteur de prélèvement, un secteur commun inférieur de colonne ainsi qu'un secteur commun supérieur de colonne, pour obtenir du monoacétal de dialdéhyde aliphatique pur en tant qu'évacuation latérale à partir du secteur de prélèvement.

11. Procédé selon l'une quelconque des revendications 1 à 10, **caractérisé en ce que** la séparation par distillation du mélange réactionnel éventuellement recuit est mise en oeuvre dans le secteur inférieur de la première colonne de distillation ou dans le secteur commun inférieur de colonne de la colonne à paroi de séparation avec ajout d'un diluant à haut point d'ébullition.

12. Procédé selon la revendication 11, **caractérisé en ce que** le diluant à haut point d'ébullition est une substance ou un mélange de substances, choisi parmi les groupes figurant ci-dessous : alcanes, substances aromatiques ou polyéthers, de préférence le groupe des polypropylèneglycols ou des polyéthylèneglycols, mieux encore le polyéthylèneglycol ayant une masse moléculaire moyenne égale à 300.
